# EUROPEAN PATENT APPLICATION

(11) **EP 1 975 616 A1**
(43) Date of publication of application: **01.10.2008**
(21) Application number: 08005629.4
(22) Date of filing: 26.03.2008
(51) Int. Cl.: G01N 33/543

(54) **Immunochromatography test tool**

(30) Priority: 29.03.2007 JP 2007087349
(71) Applicant: GC Corporation, Itabashi-ku Tokyo 174-8585 (JP)
(72) Inventor: Naito, Hiroki, Tokyo 174-8585 (JP); Nihei, Kinya, Tokyo 174-8585 (JP); Masuda, Hijiri, Tokyo 174-8585 (JP); Tachino, Atsushi, Tokyo 174-8585 (JP); Ishihara, Yoko, Tokyo 174-8585 (JP)
(74) Representative: Müller-Boré & Partner Patentanwälte

(57) **Abstract**

An objective of the present invention is to provide an immunochromatography test tool having high detection sensitivity without leakage of a test solution to a space formed between an edge of a test piece and edge of a lower cover, in an immunochromatography test tool utilizing an antigen-antibodyreaction. Theimmunochromatography test tool includes an upper cover having a test solution dropping window and a test result detection window, a lower cover having a test piece setting guide, and a test piece housed between the upper cover and the lower cover. In the test tool, contact angles of inner surfaces of the upper and lower covers are 60° to 150° with respect to water. The inner surfaces of the upper and lower covers are preferably subjected to a water repellent treatment.

## Description

The present invention relates to an immunochromatography test tool improved in detection sensitivity.

A test method utilizing an antigen-antibody reaction has been conventionally used for testing bacteria, and many methods by utilizing the antigen-antibody reaction have been proposed (for example, refertoU.S. Patent No. 5,591,645, U.S. Patent No. 4,855,240, U.S. Patent No. 4,435,504, U.S. Patent No. 4,980,298, Unexamined Japanese Patent Publication No. 61-145459, and Unexamined Japanese Patent Publication No. 6-160388). These methods are excellent in simplicity and convenience because existence or an amount of an antigen can be known only by mixing a test solution containing a collected specimen and antibody and soaking the solution into a porous membrane. These methods are generally called as an immunochromatography and the principle of the identification and quantitative determination of the immunochromatography are disclosed (for example, in Se-Hwan Paek, Seung-Hwa Lee, Joung-Hawan Cho and Young-Sang Kim, "Development of rapid One-Step Immunochromatographic assay, Methods", 22, 53-60, 2000) .

A generalimmunochromatography detects a specimen by using two antibodies. A first antibody exists in a test solution or at a portion at an end of a test piece in an approximately rectangular shape made from a porous membrane, where the test solution is dropped. This antibody is labeled with latex particles or gold colloidal particles (this antibody will be called as a labeled antibody hereinafter). When the dropped test solution includes a specimen to be detected, the labeled antibody recognizes the specimen so as to be bonded with the specimen. A complex of the specimen and labeled antibody flows by capillarity toward an absorber, which is made from a filter paper and attached to an end opposite to the end having included the labeled antibody. During the flow, the complex of the specimen and labeled antibody is recognized and caught by a second antibody (it will be called as a trapping antibody hereinafter) existing at the middle of the porous membrane and, as a result of this, the complex appears at a detection part on the porous membrane as a visible signal and is detected.

A test piece used in the above-described general immunochromatography is seted and housed between upper and lower covers called as a device cassette made of polystyrene or polypropylene, in order to easily handle the test piece. More particularly, it has an integral structure, where a test piece is put on a lower cover provided with a test piece setting guide, and closed with an upper cover provided with a dropping window for dropping a test solution to the test piece and a detection window to check the detected result appearing at the middle of the test piece made from a porous membrane.

In order to easily handle a test piece, the size of covers for housing a test piece is generally made somewhat larger than the size of the test piece, and thus a space is formed between an edge of the test piece and an edge of a lower cover. A test solution dropped to the test piece through a dropping window of an upper cover may be leaked to the space formed between the edge of the test piece and the edge of the lower cover during the flow toward an absorber in the test piece made from a porous membrane. Then, even when a test solution in an amount required for a test is dropped through a dropping window, the test solution may not flow as designed in a test piece made from a porous membrane, and thus the test solution may not be sufficiently used for the test. As a result of this, there is a problem that an error of a test increases.

An objective of the present invention is to provide an immunochromatography test tool utilizing an antigen-antibody reaction, in which the immunochromatography test tool has high detection sensitivity without leakage of a test solution to a space formed between an edge of a test piece and an edge of a lower cover.

The present inventors carried out earnest works to solve the above-described problems and, as a result, they found out the followings to complete the present invention. They paid attention to the fact that detection sensitivity can be increased by making a test solution to be absorbed in a test piece made from a porous membrane and guided to a detection part without leakage to a space formed between an edge of the test piece and an edge of a lower cover, in the process of flow of the dropped test solution toward an absorber in a test piece made from a porous membrane. Thus, they found out that the detection sensitivity can be improved by making inner surfaces of upper and lower covers to have water repellence.

That is, an aspect of the present invention is an immunochromatography test tool including an upper cover provided with a test solution dropping window and a test result detection window, a lower cover provided with a test piece setting guide, and a test piece housed between the upper cover and the lower cover, in which contact angles of inner surfaces of the upper and lower covers are 60° to 150° with respect to water. The inner surfaces of the upper and lower covers are preferably subjected to a water repellent treatment.

The immunochromatography test tool according to the present invention can guide a test solution, which is dropped through the dropping window of the upper cover to the test piece, to a detection part without leakage of the solution to a space formed between an edge of the test piece and an edge of the lower cover. Thus, detection sensitivity can be improved.

The test tool used for an immunochromatography according to the present invention will be described below. The immunochromatography test tool according to the present invention includes an upper cover provided with a test solution dropping window and a test result detection window, a lower cover provided with a test piece installing guide, and a test piece housed between the upper and lower covers.

A material of the covers constituting the immunochromatography test tool according to the present invention is not limited especially if it has hardness enough to keep the test piece. However, since the covers are thrown away after each test, a resin is proper from the viewpoint of cost, and it is preferable to use general plastics, e.g., polyethylene (PE), polypropylene (PP), polystyrene (PS), polyvinyl chloride (PVC), or polymethylmethacrylate (PMMA).

Contact angles of inner surfaces of the covers constituting the immunochromatography test tool according to the present invention are 60° to 150° with respect to water. When the contact angle is less than 60°, a test solution dropped through the dropping window of the cover to the test piece is leaked into a space formed between an edge of the test piece and an edge of the lower cover in the process of flow of the solution toward an absorber in the test piece made from a porous membrane. Thus, the test solution is not sufficiently absorbed into the porous membrane, and an error of test thereby increases. When the contact angle with respect to water is more than 150°, an incremental effect of a water repellent treatment cannot be expected. More preferably, the contact angle is 80° to 130°.

A method to make contact angles of the inner surfaces of the covers constituting the immunochromatography test tool according to the present invention to be 60° to 150° with respect to water is that roughness of the surfaces of molded covers is reduced by polishing and smoothing a surface of a mold used for producing the covers, or that the roughness of the surfaces is reduced by polishing the surface of the molded covers at the side facing a testing piece. However, when the efficiency of the treatment is considered, a method of coating a water repellant by a brush or a spray is preferable. The water repellant is not limited especially if it can give water repellence to a resin, e.g., a silicone-based, wax-based, or fluoride-based water repellant can be used.

### [Example]

### [Production of a specific antibody]

Antibodies were obtained by cultivating streptococci mutans (ATCC25175 strain) as mutans streptococci, stopping growth of the streptococci mutans with a formaldehyde aqueous solution, immunizing a mouse with the dispersion liquid itself of the streptococci mutans, and cultivating hybridomas, which were established based on a method for establishing a hybridoma by fusing cells by Kohler and Milstein, and screening the cultivated liquid by a limiting dilution method. Then the following two kinds of purified antibodies were obtained by labeling some of the obtained antibodies as mentioned below.

### [SM1 Antibody] Trapping antibody to streptococcus mutans

### [SM2 Antibody] Labeled antibody to streptococcus mutans

### [Labeling to a specific antibody]

A gold colloid having the particle diameter of 40 nm was labeled to the SM2 antibody. A commercial gold colloid (produced by British Biocell International Corporation) was diluted to have the antibody concentration of 0.1µg/mL with a phosphate buffer solution which is added with 1% bovine serum albumin (the product name: BSA, produced by Sigma Chemical Corporation) and 1% nonionic surfactant (the product name: Tween 20, produced by Sigma Chemical Corporation).

### [Production of a test piece]

A nitrocellulose membrane (the product name: Hi Flow Membrane HF076, produced by NIHON MILLIPORE K.K) was cut into a rectangle having a width of 5 mm, a length of 75 mm and a thickness of 0.4 mm to prepare a porous membrane. The SM1 antibody as a trapping antibody to streptococcus mutans was adjusted to have a concentration of 1 mg/mL with a 50mM phosphate buffer solution containing 1% bovine serum albumin. This antibody liquid was coated on the middle part of the nitrocellulose membrane orthogonally to the longitudinal direction in the amount of approximately 1 µg/cm by a micropipette to be made to soak therein. A filter paper (the product name: CFSP223, produced by NIHON MILLIPORE K. K) having a width of 5 mm, a length of 30 mm, and a thickness of 1.5 mm was fixed as an absorber so as to be overlapped with one end of the nitrocellulose membrane. Further, a propylene matrix (the product name: Quick Release Conjugate Pad, produced by NIHON MILLIPORE K.K) being 5mm by 20mm was fixed as a holder for the SM2 antibody, which was labeled with a gold colloid, at an end opposite to the absorber. Then, 30µl of the SM2 antibody labeled with a gold colloid was dropped on the matrix. Then, these were dried at 37°C for 2 hours so as to obtain a test piece. The test piece was stored in a desiccator until just before a test.

### [Production of an immunochromatography test tool]

A water-repellent spray (the product name: dufix, ultra strength waterproofing spray, produced by Henkel Japan Co., Ltd) was sprayed onto an allover surface of the inner surface of a box type cover made of materials shown in Table 1, where the box type cover was divided into an upper cover and a lower cover, the upper cover had a width of 19 mm, a length of 83 mm and a thickness of 2 mm and provided with a test solution dropping window and a test result detection window, and a lower cover had a width of 19 mm, a length of 83 mm, and a thickness of 2 mm and provided with a test piece setting guide. Then, the box typed cover was kept for 1 hour at room temperature. After that, the test piece was setted on the lower cover along the test piece setting guide provided at the lower cover, and closed with the upper cover. Thereby, an immunochromatography test tool was produced.

### [Measuring of a contact angle]

A contact angle of the inner surface of the cover with respect to water was measured by using a contact angle measuring instrument (CA-D type, produced by Kyowa Interface Science CO., Ltd). Further, the measuring was made by using a droplet process and analyzed by a θ/2 method.

### [Confirmation of an existence of an antibody reaction]

1. Streptococcus mutans solutions were prepared to make the number of streptococci mutans in a phosphate buffer solution of 50mM to be 1×10⁶ CFU/mL, 7.5×10⁵ CFU/mL, and 5.0×10⁵ CFU/mL. Then, 250 µL of these streptococcus mutans solutions were treated by a method disclosed in Unexamined Japanese Patent PublicationNo. 2002-357599. The treated solutions were used as a test solution.
2. The strength of an antibody reaction was evaluated by dropping 300µL of the testing solution into a test solution dropping window of the test tool, making the testing solution soak into a test piece, and visually checking a test result detection window after 15 minutes of soaking of the solution. The strength of an antibody reaction was evaluated by "++" which indicates a strong reaction, "+" which indicates a reaction, "±" which indicates a slight reaction, and "-" which indicates a non-reaction.

After the test, the upper cover was removed, and leakage of the test solution to a space between the edge of the test piece and the edge of the lower cover was observed.

As a comparative example, a cover not subjected to a water repellent treatment and made of polystyrene was used. By using the above-described methods, a contact angle was measured, the strength of an antibody reaction was confirmed, and leakage of a test solution was observed.

**Table 1**

| | | Example 1 | Example 2 | Example 3 | Comparative example |
|---|---|---|---|---|---|
| Materials of cover | | Polystyrene | Polyethylene | Polypropylene | Polystyrene |
| Contact Angle with respect to Water ( ° ) | | 118 | 92 | 103 | 50 |
| Strength of Antibody Reaction | 1.0×10⁶ CFU/mL | ++ | ++ | ++ | + |
| | 7.5×10⁵ CFU/mL | + | + | + | + |
| | 5.0×10⁵ CFU/mL | + | + | + | - |
| Leakage of test solution into space between edge of test piece and edge of lower cover | | Not leaked | Not leaked | Not leaked | Leaked |

Clearly from Table 1, as for Examples of which a cover inner surface was subjected to a water repellent treatment, and a contact angle with respect to water was within the range of 60° to 150°, an antibody reaction was confirmed even when the number of streptococci mutans was as low as 5.0×105 CFU/mL, and a test solution was not leaked. On the other hand, as for Comparative example of which a cover inner surface was not subjected to a water repellent treatment and a contact angle with respect to water was 50°, a test solution was leaked, an antibody reaction was not confirmed when the number of streptococci mutans was 5.0×105 CFU/mL. Therefore, it is found out that, when an immunochromatography test tool has a cover inner surface subjected to a water repellent treatment and has a contact angle with respect to water within the range of 60° to 150°, a dropped test solution can be guided to a detection part of a test piece without leakage of the solution, and thus the detection sensitivity is high.

## Claims

1. An immunochromatography test tool comprising:
an upper cover provided with a test solution dropping window and a test result detection window,
a lower cover provided with a test piece setting guide, and
a test piece housed between the upper cover and the lower cover,
wherein contact angles of inner surfaces of the upper and lower covers are 60° to 150° with respect to water.

2. The immunochromatography test tool as claimed in claim 1,
wherein the inner surfaces of the upper and lower covers are subjected to a water repellent treatment.
